(19) Europäisches Patentamt
European Patent Office
Office européen des brevets

(11) Veröffentlichungsnummer: **0 000 362**
**B1**

(12) # EUROPÄISCHE PATENTSCHRIFT

(21) Anmeldenummer: **78100284.5**

(22) Anmeldetag: **30.06.78**

(51) Int. Cl.³: **C 07 C 125/00,**
C 07 C 125/03

(54) **Alpha-Halogenalkylcarbamidsäurehalogenide und Verfahren zu deren Herstellung.**

(30) Priorität: **16.07.77 DE 2732284**
**17.09.77 DE 2741980**

(43) Veröffentlichungstag der Anmeldung:
**24.01.79 Patentblatt 79/02**

(45) Bekanntmachung des Hinweises auf die Patenterteilung:
**03.09.80 Patentblatt 80/18**

(84) Benannte Vertragsstaaten:
**BE CH DE FR GB NL**

(56) Entgegenhaltungen:
**The Journal of Organic Chemistry**
**Band 28(7) Seiten 1825—1830 (1963)**

(73) Patentinhaber: **BASF Aktiengesellschaft**
**Carl-Bosch-Strasse 38**
**D-6700 Ludwigshafen (DE)**

(72) Erfinder: **Koenig, Karl-Heinz, Dr.**
**Pierstrasse 8a**
**D - 6710 Frankenthal (DE)**
**Reitel, Christian, Dr.**
**Kleingemuender Strasse 75**
**D - 6900 Heidelberg (DE)**
**Mangold, Dietrich, Dr.**
**Hermann-Walker-Strasse 49**
**D - 6930 Neckargemuend (DE)**

**EP 0 000 362 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Alpha-Halogenalkylcarbamidsäurehalogenide und Verfahren zu deren Herstellung

Die Erfindung betrifft ein Verfahren zur Herstellung von $\alpha$-Halogenalkylcarbamidsäurehalogeniden durch Umsetzung von Vinylisocyanat oder N-tert.Alkyl-N-(1-alkenyl)carbamidsäurehalogeniden mit Halogenwasserstoff bei −78°C bis +80°C.

Es ist aus der Angewandten Chemie, Band 74 (1962), Seiten 848 bis 855 bekannt, daß man Alkylcarbamidsäurechloride mit elementarem Chlor in die entsprechenden $\alpha$-Chloralkylcarbamidsäurechloride umsetzt. Die dabei anfallenden Produkte sind jedoch Gemische sowohl mit Bezug auf den halogenierungsgrad wie auf die Stellung der eintretenden Halogenatome. Das Verfahren ist im Hinblick auf Ausbeute und Reinheit des Endstoffs sowie einfachen und wirtschaftlichen Betrieb nicht befriedigend.

Es werden nun die neuen $\alpha$-Halogenalkylcarbamidsäurehalogenide der Formel

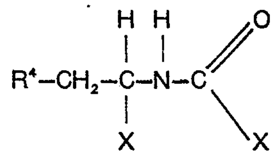

I

worin $R^4$ ein Wasserstoffatom oder einen Alkylrest mit 1 bis 20 Kohlenstoffatom bedeutet und X ein Halogenatom bezeichnet, gefunden.

Es wurde weiterhin gefunden, daß man $\alpha$-Halogenalkylcarbamidsäurehalogenide der Formel

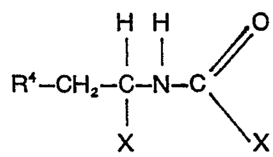

I

worin $R^4$ ein Wasserstoffatom oder einen Alkylrest mit 1 bis 20 Kohlenstoffatomen bedeutet und X ein Halogenatom bezeichnet, vorteilhaft erhält, wenn man Vinylisocyanat oder N-tert.Alkyl-N-(1-alkenyl)-carbamidsäurehalogenide der Formel

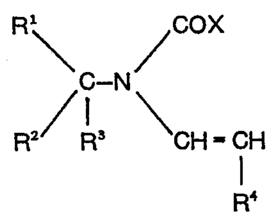

II

worin $R^4$ ein Wasserstoffatom oder einen Alkylrest mit 1 bis 20 Kohlenstoffatomen bedeutet und X ein Halogenatom bezeichnet, die Reste $R^1$, $R^2$ und $R^3$ gleich oder verschieden sein können und jeweils Alkylreste bedeuten, mit Halogenwasserstoff bei einer Temperatur von −78°C bis +80°C umsetzt.

Weiterhin wurden die neuen $\alpha$-Halogenäthylcarbamidsäurehalogenide, insbesondere das neue $\alpha$-Chloräthylcarbamidsäurechlorid, gefunden.

Die Umsetzung kann im Falle der Verwendung von Vinylisocyanat und Chlorwasserstoff durch die folgenden Formeln wiedergegeben werden:

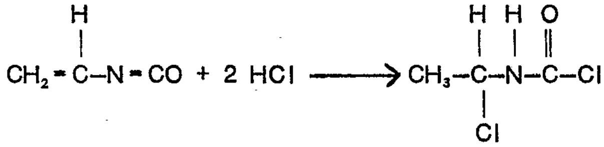

Im Falle der N-tert.Alkyl-N-(1-alkenyl)-carbamidsäurehalogenide, z.B. der Chloride, tritt entsprechend der Reaktionsgleichung quantitativ ein Fragmentierung in tert. Alkylchlorid und $\alpha$-Chloräthylcarbamidchlorid ein.

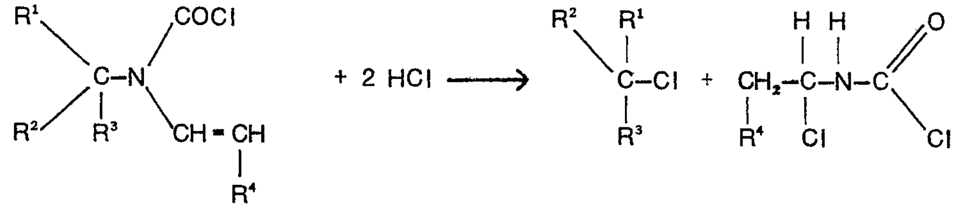

Im Hindblick auf das bekannte Verfahren liefert das Verfahren nach der Erfindung auf einfacherem und wirtschaftlicherem Wege $\alpha$-Halogenalkylcarbamidsäurehalogenide in besserer Ausbeute und Reinheit. Die Aufarbeitung ist wesentlich einfacher, da kein komponentenreiches Reaktionsgemisch erhalten wird.

Die Umsetzung von tert.Alkyl-N-(1-alkenyl)-carbamidsäurehalogeniden, insbesondere den Chloriden, mit Halogen-wasserstoff, insbesondere Chlorwasserstoff, gestattet es, in besonders reiner Form und unter schonenden Reaktionsbedingungen das thermostabile Halogenid, insbesondre $\alpha$-Chloralkylcarbamidsäurechlorid, herzustellen, wohingegegen bei der Einwirkung von elementarem Halogen auf Alkylcarbamidsäurechloride (Ang., loc. cit.) Produktgemische hinsichtlich der Stellung des eintretenden Halogenatoms sowie des Halogenierungsgrades erhalten werden. Das bei der Reaktion gleichzeitig anfallende tert.Alkylhalogenid ist unter den üblichen Reaktionsbedindungen inert und muß daher in der Regel bei den weiteren Umsetzungen des $\alpha$-Chloralkylcarbamidsäurechlorids nicht entfernt werden.

Alle diese vorteilhaften Ergebnisse sind überraschend, denn angesichts der sehr reaktionsfreudigen Ausgangsstoffe war mit der Bildung verschiedenartiger Reaktionsprodukte zu rechnen. Auch war zu vermuten, daß $\alpha,\beta$-ungesättigte Stickstoffverbindungen sehr leicht unter der Einwirkung von Säuren polymerisieren oder hydrolysieren. So geht z.B. N-Vinylpyrrolidon durch Einwirkung schon geringer Mengen an anorganischen Säuren in ein Gemisch von Oligomeren über (Ullmanns Encyclopädie der technischen Chemie, Band 14, Seite 261). Bull. Soc. Chim. Belg., Band 65, Seiten 291 bis 296 (1956) zeigt, daß Vinylisocyanat mit wäßriger 12-N-Salzsäure in Aceton zu Acetaldehyd hydrolysiert.

Vinylisocyanat kann z.B. durch Umsetzung von Acrylsäurechlorid mit Natriumazid (Bull., loc. cit.) oder durch thermische Zerlegung von N-tert.-Butyl-N-vinylcarbamidsäurechlorid hergestellt werden. Man verwendet den Halogenwasserstoff, vorteilhaft Bromwasserstoff und insbesondere Chlorwasserstoff, in stöchiometrischer Menge oder im Überschuß, vorzugsweise in einer Menge von 2 bis 2,2 Mol Halogenwasserstoff je Mol Vinylisocyanat oder N-tert.Alkyl-N-(1-alkenyl)-carbamidsäurehalogenid.

Als tertiäre Alkylreste, $R^1$, $R^2$ und $R^3$, die gleich oder verschieden sein können, solche mit 4 bis 20 insbesondere 4 bis 12 Kohlenstoffatomen, in Betracht. Insbesondere sind der tert.Butyl- und tert.Amylrest zu nennen. Demgemäß können die Reste $R^1$ bis $R^3$ in der angegebenen Formel Alkylreste mit 1 bis 6 Kohlenstoffatomen, insbesondre Methyl und Äthyl bedeuten. $R^4$ bezeichnet vorteilhaft Wasserstoff oder einen Alkylrest mit 1 bis 20, insbesondere 1 bis 12, vorzugsweise 1 bis 6 Kohlenstoffatomen; bevorzugt sind Wasserstoff, Methyl und Äthyl.

Die Umsetzung wird bei einer Temperatur von −78°C bis +80°C, vorteilhaft von +40°C bis −78°C, vorzugsweise im Falle von Vinylisocyanat von +30°C bis −78°C, insbesondere bei 0°C bis −40°C, vorzugsweise im Falle von N-tert.Alkyl-N-(1-Alkenyl)-carbamidsäurehalogeniden von −10°C bis 20°C, drucklos oder unter Druck, vorzugsweise bei 0,7 bis 2 bar, kontinuierlich oder diskontinuierlich durchgeführt. Man kann ohne Lösungsmittel unsetzen, zweckmäßig verwendet man aber unter den Reaktionsbedingungen inerte Lösungsmittel, Wasser wird in Falle von Vinylisocyanat nicht verwendet. Wegen der Reaktivität der entstehenden $\alpha$-Halogenalkylcarbamidsäurehalogenide arbeitet man bevorzugt wasserfrei; prinzipiell kann man aber auch mit wäßriger Salzasäure arbeiten. Bevorzugt arbeitet man in Lösungsmitteln, die bei der weiteren Umsetzung des Endsstoffs, insbesondere des $\alpha$-Chloräthylencarbamidsäurechlorids, als Reaktionsmedium Verwendung finden. Als Lösungsmittel kommen z.B. in Frage: aromatische Kohlenwasserstoffe, z.B. Toluol, Äthylbenzol, o-, m-, p-Xylol, Isopropylbenzol, Methylnaphthalin, aromatische Äther; Halogenkohlenwasserstoffe, insbesondere Chlorkohlenwasserstoff, z.B. Tetrachloräthylen, 1,1,2,2- oder 1,1,1,2-Tetrachloräthan, Amylchlorid, Cyclohexylchlorid, 1,2,-Dichlorpropan, Methylenchlorid, Dichlorbutan, Isopropylbromid, n-Propylbromid, Butylbromid, Chloroform, Äthyljodid, Propyljodid, Chlornaphthalin, Dichlornaphthalin, Tetrachlorkohlenstoff,1,1,1- oder 1,1,2-Trichloräthan, Trichloräthylen, Pentachloräthyn, 1,2-Dichloräthan, 1,1-Dichloräthan, n-Propylchlorid, 1,2-cis-Dichloräthylen, n-Butylchlorid, 2-, 3- und iso-Butylchlorid, Chlorbenzol, Fluorbenzol, Brombenzol, Jodbenzol, o-, p- und m-Dichlorbenzol, o-, p-, m-Dibrombenzol, o-, m-, p-Chlortoluol, 1,2,4-Trichlorbenzol, 1,10-Dibromdekan, 1,4-Dibrombutan; Äther, z.B. Äthylenpropyläther, Methyl-tert.-butyläther, n-Butyläther, Di-n-butyläther, Diisobutyläther, Diisoamyläther, Diisopropyläther, Anisol, Phenetol, Cyclohexylmethyläther, Diäthyläther, Äthylenglykoldimethyläther, Tetrahydrofuran, Dioxan, Thianisol, $\beta,\beta'$-Dichlordiäthyläther; Ketone wie Methyläthylketon, Aceton, Diisopropylketon, Diäthylketon, Methylisobutylketon, Mesityloxid, Acetophenon, Cyclohexanon, Äthylisoamylketon, Diisobutylketon, Methylcyclohexanon, Dimethylcyclohexanon; Ester wie Methylacetat, n-Propylacetat, Methylpropionat, Butylacetat, Äthylformiat, Phthalsäuremethylester, Benzoesäuremethylester, Essigester, Phenylacetat und höher siedende Ester; aliphatische oder cycloaliphatische Kohlenwasserstoff, z.B. Pentan, Heptan, Pinan, Nonan, Benzinfraktionen innerhalb eines Siedepunktintervalls von 70 bis 190°C, Cyclohexan, Methylcyclohexan, Dekalin, Petroläther, Hexan, Ligroin, 2,2,4-Trimethylpentan, 2,2,3-Trimethylpentan, 2,3,3,-Trimethylpentan, Octan; und entsprechende Gemische. Zweckmäßig verwendet man das Lösungsmittel in einer Menge von 200 bis 10 000 Gewichtsprozent, vorzugsweise von 300 bis 2 000 Gewichtsprozent, bezogen auf den Ausgangsstoff Vinylisocyanat.

Die Konzentration der Lösungen der N-tert.Alkyl-N-(1-alkenyl)-carbamidsäurechloride ist in weiten Grenzen variabel. Bevorzugt wird ein Konzentrationsbereich zwischen 1 und 50 Gewichtsprozent angewandt.

Die Reaktion kann wie folgt durchgeführt werden: Ein Gemisch der Ausgangsstoffe und zweckmäßig Lösungsmittel wird während 0,1 bis 4 Stunden bei der Reaktionstemperatur gehalten. Vinylisocyanat wird vorteilhaft im Lösungsmittel vorgelegt und bei der Reaktionstemperatur mit dem Halogenwasserstoff begast. Zweckmäßig rührt man die Reaktionslösung 0,25 Stunden bis ein Stunde nach. Das N-tert.Alkyl-N-(1-alkenyl)-carbamidsäurehalogenid wird zweckmäßig in einem inerten Lösungsmittel vorgelegt und bei einer Temperatur von z.B. —10°C bis 0°C mit Halogenwasserstoff begast. Nach Beendigung der Umsetzung rührt man die Reaktionslösung einige Zeit, z.B. 15 Minuten, nach und bläst überschüssigen Halogenwasserstoff mittels $N_2$ aus. Dann wird der Endstoff aus dem Gemisch in üblicher Weise, z.B. durch Kristallisation und Filtration, abgetrennt.

Die nach dem Verfahren der Erfindung hergestellten $\alpha$-Halogenalkylcarbamidsäurehalogenide, insbesondere das $\alpha$-Chloräthylcarbamidsäurechlorid, sind wertvolle Ausgangsstoffe für die Herstellung von Lackrohstoffen, Textilbeschichtungsmitteln, Farbstoffen, Pharmaceutica und Pflanzenschutzmitteln.

Die in den Beispielen genannten Teile sind Gewichtsteile.

## Beispiel 1

69 Teile Vinylisocyanat werden in 250 Teilen Tetrachlorkohlenstoff vorgelegt. In diese Lösung werden bei —35°C innerhalb einer Stund 73 Teile Chlorwasserstoff eingeleitet. Die Reaktionslösung wird weitere 15 Minuten bei dieser Temperatur gerührt. Man erhält nach Filtration 137 Teile (95% der Theorie) $\alpha$-Chloräthylcarbamidsäurechlorid vom Fp. 21°C und NMR-Spektrum in $CCl_4$ (Standard Tetramethylsilan).

| | |
|---|---|
| ($CH_3$—) | 1,8 ppm |
| (Cl—C—H) | 5,8 ppm |
| (NH) | 7,5 ppm. |

## Beispiel 2

161,5 Teile N-tert.butyl-N-vinylcarbamidsäurechlorid werden bei 0°C vorgelegt und hierzu innerhalb 60 Minuten 75 Teile Chlorwasserstoff eingegast. Die Reaktionsmischung wird 15 Minuten bei dieser Temperatur nachgerührt und überschüssiger HCl mit $N_2$ ausgeblasen. Tert.Butylchlorid wird bei vermindertem Druck abgezogen und das $\alpha$-Chloräthylcarbamidsaürechlorid in $CCl_4$ umkristallisiert. 136 Teile (95% der Theorie); Fp. 20/21°C.

## Beispiel 3

175,5 Teile N-tert.Amyl-N-vinylcarbamidsäurechlorid werden bei 10°C vorgelegt und unter Rühren 75 Teile HCl eingeleitet.

Nach Beendigung der Umsetzung wird die Reaktionsmischung noch 30 Minuten bei Raumtemperatur nachgerührt und überschüssiger HCl mit $N_2$ ausgeblasen.

Das tert. Amylchlorid wird bei vermindertem Druck abgezogen.

Es hinterbleiben 130 Teile (91,5% der Theorie) $\alpha$-Chloräthylcarbamidsäurechlorid vom Fp. 20°C.

## Beispiel 4

50 Teile Vinylisocanat werden in 150 Teilen Dichlormethan vorgelegt. Innerhalb von 70 Minuten werden in diese Lösung 125 Teile Bromwasserstoff bei —20°C eingeleitet. Die Reaktionslösung wird weitere 40 Minuten bei dieser Temperatur gerührt. Nach Filtration erhält man 150 Teile (90% der Theorie) $\alpha$-Bromäthylcarbamidsäurebromid vom Fp. 55°C und NMR-Spektrum in $CDCl_3$ (Standard Tetramethylsilan).

| | |
|---|---|
| $CH_3$—) | 2.0 ppm |
| (Br—C—H) | 5,9 ppm |
| (NH) | 6,8 ppm |

**Patentansprüche**

1. α-Halogenalkylcarbamidsäurehalogenide der Formel

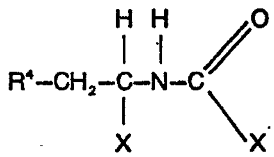

I

worin $R^4$ ein Wasserstoffatom oder einen Alkylrest mit 1 bis 20 Kohlenstoffatomen bedeutet und X ein Halogenatom bezeichnet.

2. Verfahren zur Herstellung von α-Halogenalkylcarbamidsäurehalogeniden der Formel

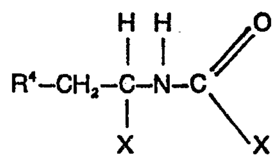

I

worin $R^4$ ein Wasserstoffatom oder einen Alkylrest mit 1 bis 20 Kohlenstoffatomen bedeutet und X ein Halogenatom bezeichnet, dadurch gekennzeichnet, daß man Vinylisocyanat oder N-tert.Alkyl-N-(1-alkenyl)-carbamid-säurehalogenide der Formel

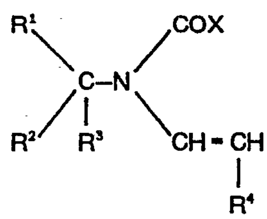

II

worin $R^4$ ein Wasserstoffatom oder einen Alkylrest mit 1 bis 20 Kohlenstoffatomen bedeutet und X ein Halogenatom bezeichnet, die Reste $R^1$, $R^2$ und $R^3$ gleich oder verschieden sein können und jeweils Alkylreste bedeuten, mit Halogenwasserstoff bei einer Temperatur von −78°C bis +80°C umsetzt.

**Revendications**

1. Halogénures de carbamyle α-halo-alcoyl-substitués de la formule générale

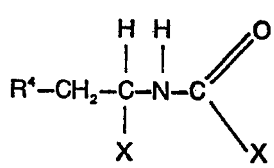

I

dans laquelle le symbole $R^4$ représente un atome d'hydrogène ou un radical alcoyle en $C_1$ à $C_{20}$ et le symbole X un atome d'halogène.

2. Procédé de préparation d'un halogénure de carbamyle α-halo-alcoyl-substitué de la formule générale

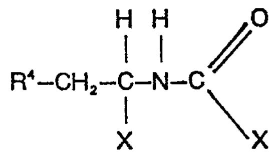

I

dans laquelle le symbole $R^4$ représente un atome d'hydrogène ou un radical alcoyle en $C_1$ à $C_{20}$ et le symbole x un atome d'halogène, caractérisé en ce que l'on fait réagir l'isocyanate de vinyle ou un halogénure de N-tertio-alcoyl-N-(1-alcényl)-carbamyle de la formule générale

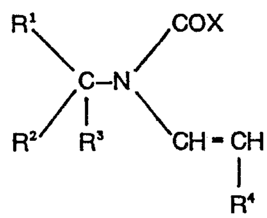

II

dans laquelle les symboles $R^4$ et X possèdent les significations définies et les symboles $R^1$, $R^2$ et $R^3$, qui peuvent être identiques ou différents, désignent chacun un radical alcoyle, entre $-78°C$ et $+80°C$ avec un hydracide halogéné.

3. Halogénure de $\alpha$-halo-éthyl-carbamyle.
4. Chlorure de $\alpha$-chlor-éthyl-carbamyle.

**Claims**

1. $\alpha$-Haloalkylcarbamyl halides of the formula

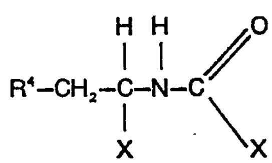

I

where $R^4$ denotes hydrogen or alkyl of 1 to 20 carbon atoms and X denotes halogen.

2. A process for the production of $\alpha$-haloalkylcarbamyl halides of the formula

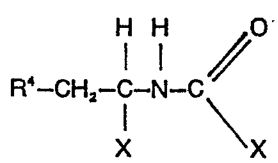

I

where $R^4$ denotes hydrogen or alkyl of 1 to 20 carbon atoms and X denotes halogen, characterized in that vinyl isocyanate or a N-tert-alkyl-N-(1-alkenyl)-carbamyl halide of the formula

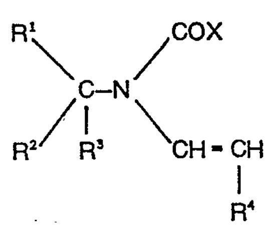

II

where $R^4$ denotes hydrogen or alkyl of 1 to 20 carbon atoms, X denotes halogen, and the radicals $R^1$, $R^2$ and $R^3$ are identical or different and denote alkyl radicals, is reacted with a hydrogen halide at a temperature of −78°C to +80°C.

3. $\alpha$-Haloethylcarbamyl halides.

4. $\alpha$-Chloroethylcarbamyl chloride.